(19) Europäisches Patentamt
European Patent Office
Office européen des brevets

(11) **EP 3 517 963 A1**

(12) **EUROPEAN PATENT APPLICATION**
published in accordance with Art. 153(4) EPC

(43) Date of publication:
**31.07.2019 Bulletin 2019/31**

(21) Application number: **17853476.4**

(22) Date of filing: **22.09.2017**

(51) Int Cl.:
***G01N 33/68*** (2006.01)     ***G01N 33/52*** (2006.01)
***G01N 33/543*** (2006.01)     ***G01N 21/17*** (2006.01)

(86) International application number:
**PCT/KR2017/010499**

(87) International publication number:
**WO 2018/056762 (29.03.2018 Gazette 2018/13)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **22.09.2016 KR 20160121694**
            **21.09.2017 KR 20170122084**

(71) Applicant: **DxGen Corp.**
**Gunpo-si, Gyeonggi-do 15807 (KR)**

(72) Inventors:
• **LEE, Jin Woo**
  **Suwon-si**
  **Gyeonggi-do 16324 (KR)**
• **CHEON, Seon Ah**
  **Seoul 06920 (KR)**

(74) Representative: **Ter Meer Steinmeister & Partner**
**Patentanwälte mbB**
**Nymphenburger Straße 4**
**80335 München (DE)**

(54) **REAGENT COMPOSITION FOR MEASURING GLYCATED ALBUMIN AND METHOD FOR MEASURING GLYCATED ALBUMIN USING SAME**

(57) The present invention relates to a reagent composition for measuring glycated albumin to diagnose the presence or absence of diabetes and a method of measuring glycated albumin using the same, and more particularly to a reagent composition for measuring glycated albumin, the composition including a dye-encapsulated silica nanoparticle-boronic acid, and to a method of measuring glycated albumin using the same. The method of measuring glycated albumin according to the present invention includes (a) introducing blood or a plasma solution into a reagent including a "dye-encapsulated silica nanoparticle-boronic acid" specifically binding to glycated albumin, followed by reaction, (b) injecting a reactant into an absorption pad of a cartridge, followed by washing with a washing liquid, (c) measuring an optical reflectance of the absorption pad using an optical instrument to measure an amount of glycated albumin, (d) introducing blood or a plasma solution into a reagent including a dye specifically binding to total albumin, followed by reaction, (e) injecting a reactant into an absorption pad of a cartridge, followed by washing with a washing liquid, (f) measuring an optical reflectance of the absorption pad using the optical instrument to measure an amount of total albumin, and (g) calculating a ratio of glycated albumin on the basis of the measured amounts of glycated albumin and total albumin. In the reagent composition for measuring glycated albumin according to the present invention, since a dye is encapsulated in silica nanoparticles, the inherent absorption wavelength of the dye is not affected by pH and the composition has excellent stability even when stored for one month or more.

【Fig. 3】

## Description

## Technical Field

The present invention relates to a reagent composition for measuring glycated albumin to diagnose the presence or absence of diabetes and a method of measuring glycated albumin using the same, and more particularly to a reagent composition for measuring glycated albumin, the composition including a dye-encapsulated silica nanoparticle-boronic acid, and to a method of measuring glycated albumin using the same.

## Background Art

Diabetes is a metabolic disease caused by an abnormality of insulin, which plays a role in regulating blood sugar. It is classified into Type 1 diabetes, which occurs due to insulin deficiency when insulin-producing cells are destroyed due to an abnormality in the immune system, and Type 2 diabetes, caused by a lack of insulin secretion or an ineffective use of insulin, depending on the cause of a disease.

Diabetes is characterized by hyperglycemia, in which the glucose concentration in the blood is elevated. Failure to control blood glucose may lead to complications such as diabetic retinopathy, kidney disease, and foot lesions. Therefore, the importance of blood glucose management for diabetics is increasing.

Conventional diabetes measurement markers use glucose. However, since blood glucose fluctuates greatly before and after a meal, there are problems in that error due to the measurement time and fluctuation due to the condition of the patient may be very evident. Further, measurement of glucose oxidase, used for glucose measurement, may be vulnerable to environmental influences such as pH or other interfering materials contained in the blood, and hydrogen peroxide may be generated, thus affecting enzyme activity.

Therefore, recently, glycated hemoglobin (HbA1c) has been used as a biomarker for measuring a blood glucose level, which enables more accurate measurement of blood glucose levels and is more stable that glucose. Once glycated hemoglobin is generated, it is stable until erythrocytes disappear. Therefore, since glycated hemoglobin is used as an indicator for showing the mean blood glucose level over 2 to 3 months, it is used to diagnose and investigate the progress of diabetes treatment in practice. However, it is known that a glycated-hemoglobin measurement method is not suited to patients of some diseases that make it difficult to maintain constant blood glucose, as in terminal chronic renal failure or patients with erythrocyte abnormalities.

Albumin is a protein that is present not only in the blood but also in major organs and body fluids, and glycated albumin, in which albumin binds to glucose, may be formed depending on the glucose concentration in the blood. Since albumin has a glucose-binding rate that is about 10 times higher than that of hemoglobin, glycated albumin is more sensitive to changes in blood glucose than glycated hemoglobin. Since the lifetime of albumin is short, roughly 15 to 20 days compared to about 90 days, which is the lifespan of an erythrocyte, the average level of glucose in the blood for the last two weeks may be monitored thereby. Therefore, albumin is useful as an important blood glucose control indicator in patients of terminal chronic renal failure, which makes it difficult to maintain constant blood glucose, patients with iron deficiency anemia, and diabetic patients with variant hemoglobin.

U.S. Patent Nos. 7871789, 6008006, and 8507223, and Chinese Patent Publication Nos. 104673878 and 104614459 relate to a conventional method of measuring glycated albumin. The patents disclose a glycated-albumin enzymatic method in which the glycated protein present in a sample is decomposed into a glycated amino acid or a glycated peptide using a protease and in which hydrogen peroxide ($H_2O_2$), generated due to an enzymatic reaction of glycated-amino-acid oxidase series (EC 1.5.3) that specifically react therewith, is then measured using a peroxidase enzyme.

The glycated-albumin enzymatic method has high selectivity and accuracy for albumin and can perform tests within a shorter time (10 to 30 minutes) than in the immunoassay. However, since the activity of the enzyme protein greatly influences the efficiency of the glycated albumin assay method due to the nature thereof, strict caution is required for the maintenance and storage of enzyme activity.

The glycated-albumin enzymatic method is a complex measurement method including multiple stages, for example, a step for removing the glycated amino acid and the glycated peptide already present in the sample must first be performed, and a step of decomposing a glycated protein into a glycated amino acid or peptide using a protease must also be performed in advance because the glycated amino acid oxidase (EC 1.5.3) cannot use the intact glycated protein as a substrate. Therefore, it is necessary to develop a method of measuring glycated albumin more simply and quickly.

Further, U.S. Patent Nos. 5223392 and 5908925, European Patent Publication No. 0657470, European Patent No. 0257421, and Chinese Patent Publication No. 103554256 disclose an enzyme immunoassay (ELISA) using an albumin antibody and a glycated albumin antibody conjugated with peroxidase in order to detect glycated albumin. Ikeda et al. disclose an enzyme-boronic acid immunoassay (ELIBA) using an albumin antibody and boronic acid conjugated with peroxidase (Ikeda et al, Clin Chem. 44(2):256-263, 1998). Although these methods show high selectivity and accuracy, the methods require the storage and maintenance of antibodies and enzymes and moreover take a lot of time (30 to 90 minutes), which imposes a limitation on the development of rapid kits.

With respect to POC (point of care) or such rapid kits, U.S. Patent No. 9128085, U.S. Patent Publication No. 2006-0270060, U.S. Patent Publication No. 2008-0227210, U.S. Patent Publication No. 2010-0167306, U.S. Patent No. 5470759, and U.S. Patent No. 7659107 disclose a method adopting disposable strips and cassettes using antibody-based lateral flow immunochromatography in order to measure albumin and glycated albumin in samples such as blood and saliva. U.S. Patent Publication No. 2014-0170766 discloses a method of manufacturing a rapid kit adopting lateral flow immunochromatography using an albumin aptamer and a glycated albumin aptamer derived from a nucleic acid having action similar to an antibody. U.S. Patent Publication No. 2014-0335630 discloses a measurement method using a glycated albumin aptamer and SPR (surface plasmon resonance). However, like antibodies, attention must also be paid to the maintenance and storage of aptamers including antibodies or nucleic acids. Therefore, it is necessary to develop diagnostic reagents and methods capable of measuring glycated albumin more stably and quickly.

Meanwhile, a boronic acid-affinity method is widely used for the detection of glycated hemoglobin. U.S. Patent Nos. 5631364 and 7374943 and International Patent No. 2014-033258 disclose a method that includes reacting a dye-binding boronic acid derivative with glycated hemoglobin in the blood, loading the resultant substance on a cartridge including a porous filter paper, performing washing, and measuring the reflectances (%) of total hemoglobin and dye-binding glycated hemoglobin, thereby determining the ratios of the two materials.

Further, U.S. Patent No. 5589393 discloses a method of measuring glycated hemoglobin by fixing a boronic acid derivative on agarose beads to improve safety and by reacting the resultant substance with glycated hemoglobin in the blood using a disposable cartridge. U.S. Patent No. 8557590 and Korean Patent No. 1128037 disclose a method of measuring glycated hemoglobin, in which a reacted sample is directly loaded on a cartridge including a porous filter paper.

Although a glycated hemoglobin rapid kit employing a boronic acid-affinity method has been studied, a glycated albumin rapid kit employing a boronic acid-affinity method has not yet been developed. This is because there is a limitation in applying the boronic acid-affinity method to the measurement of glycated albumin. First, xylene cyanol-DAPOL-boronic acid (xylene cyanol-DAPOL-CPBA), which is a dye-boronic acid derivative mainly used for the measurement of glycated hemoglobin, has an absorption wavelength of 620 nm, which is the same as that of bromocresol green or bromocresol purple, used to measure albumin, which is not suitable for measuring glycated albumin. Therefore, it is necessary to develop a boronic acid derivative binding to a dye distinguished from bromocresol green or purple for glycated albumin measurement.

As a result of efforts made to solve the above problems, the inventors of the present invention have found that when using boronic acid conjugated with dye-encapsulated silica nanoparticles including (a) a dye specifically binding to total albumin and (b) a dye that is complementary in color to the dye specifically binding to total albumin, the amounts of albumin and glycated albumin are capable of being measured quickly and accurately in a simple and stable manner using an optical instrument, whereby the present invention has been completed.

**Disclosure**

**Technical Problem**

An object of the present invention is to provide a reagent composition for measuring glycated albumin to simply and accurately diagnose the presence or absence of diabetes, and a method of measuring glycated albumin using the same.

**Technical Solution**

In order to accomplish the above object, the present invention provides a reagent composition for measuring glycated albumin. The reagent composition includes (a) a dye specifically binding to total albumin, and (b) a "dye-encapsulated silica nanoparticle-boronic acid" specifically binding to glycated albumin.

The present invention also provides a method of measuring glycated albumin. The method includes (a) introducing blood or a plasma solution into a reagent including a "dye-encapsulated silica nanoparticle-boronic acid" specifically binding to glycated albumin, followed by reaction, (b) injecting a reactant into an absorption pad of a cartridge, followed by washing with a washing liquid, (c) measuring the optical reflectance of the absorption pad using an optical instrument to measure the amount of glycated albumin, (d) introducing blood or a plasma solution into a reagent including a dye specifically binding to total albumin, followed by reaction, (e) injecting a reactant into an absorption pad of a cartridge, followed by washing with a washing liquid, (f) measuring the optical reflectance of the absorption pad using the optical instrument to measure the amount of total albumin, and (g) calculating the ratio of glycated albumin on the basis of the measured amounts of glycated albumin and total albumin.

In the present invention, the dye specifically binding to the total albumin is bromocresol green or bromocresol purple.

In the present invention, a dye encapsulated in silica nanoparticles is a yellow-colored dye or a red-colored dye, the yellow-colored dye has an absorption wavelength of 400 to 430 nm, and the red-colored dye has an absorption wavelength of 500 to 530 nm.

In the present invention, the yellow-colored dye is tartrazine and the red-colored dye is red 80.

In the present invention, "dye-encapsulated silica nanoparticles" are manufactured by adding a dye and silica to a mixture solution of water and a surfactant or a mixture solution of water and an organic solvent, followed by agitation and addition of a basic catalyst, and specifically bind to the glycated albumin.

In the present invention, the diameter of "dye-encapsulated silica nanoparticles" is 10 to 500 nm.

In the present invention, the "dye-encapsulated silica nanoparticle-boronic acid" is manufactured by aminating "dye-encapsulated silica nanoparticles", followed by conjugation with 4-carboxylicphenyl boronic acid (CPBA), or by carboxylating the "dye-encapsulated silica nanoparticles", followed by conjugation with 3-aminophenylboronic acid (APBA).

In the present invention, the optical instrument simultaneously radiates a wavelength of a dye specifically binding to the total albumin and a specific wavelength of the "dye-encapsulated silica nanoparticle-boronic acid" as light sources, thus measuring the optical reflectance.

In the present invention, in the method of measuring the glycated albumin, diabetes is diagnosed according to the ratio of the glycated albumin.

## Advantageous Effects

Since a reagent composition for measuring glycated albumin according to the present invention includes a "dye-encapsulated silica nanoparticle-boronic acid", the inherent absorption wavelength of the dye is not affected by pH and the composition has excellent stability even when stored for one month or more. A plurality of dye molecules is encapsulated in a single silica nanoparticle, so that the amount of light absorbed by one particle is larger than that absorbed by one dye molecule. Accordingly, it is possible to accurately measure the amount of glycated albumin in the blood, which conventionally has a low detection limit.

## Description of Drawings

FIG. 1 is a view showing a method of manufacturing "dye-encapsulated silica nanoparticles" according to an embodiment of the present invention;

FIG. 2 is a view showing a binding reaction of a "dye-encapsulated silica nanoparticle-boronic acid" and glycated albumin according to the embodiment of the present invention;

FIG. 3 is a flowchart showing a method of measuring glycated albumin and total albumin using a reagent composition for measuring glycated albumin of the present invention and an optical instrument;

FIG. 4 is a graph showing the absorbance depending on the absorption wavelength of the "dye-encapsulated silica nanoparticle-boronic acid" manufactured according to the embodiment of the present invention and the absorption wavelength of each of the dyes that are used;

FIG. 5 is a graph showing (A) the shape image of the "dye-encapsulated silica nanoparticle-boronic acid" manufactured according to the embodiment of the present invention, measured using a scanning electron microscope, and (B) the size thereof, analyzed using a dynamic light-scattering method; and

FIG. 6 is a graph showing the reflectance value of the "dye-encapsulated silica nanoparticle-boronic acid" depending on the glycated albumin concentration of a plasma sample, the concentration of which is known.

## Best Mode

In the present invention, the intention is to confirm that when using boronic acid conjugated with dye-encapsulated silica nanoparticles including (a) a dye specifically binding to total albumin and (b) a dye that is complementary in color to the dye specifically binding to total albumin, the amounts of albumin and glycated albumin are capable of being measured accurately in a simple and stable manner using an optical instrument.

In the present invention, a "dye-encapsulated silica nanoparticle-boronic acid" specifically binding to glycated albumin was manufactured, and a dye specifically binding to total albumin was added thereto, thus manufacturing a reagent composition for measuring glycated albumin. Next, blood or a plasma sample containing albumin and glycated albumin was added to the manufactured reagent composition to perform reaction, and was then injected into an absorption pad, followed by washing of the unreacted dye and the dye binding to impurities. The optical reflectance of the absorption pad was then measured using the optical instrument in order to measure the amounts of albumin and glycated albumin. As a result, it was confirmed that it was possible to diagnose diabetes simply and quickly using the ratio of glycated albumin.

That is, in an embodiment of the present invention, a yellow-colored tartrazine dye was encapsulated in silica nanoparticles, the hydroxyl group (-OH) on the surface thereof was substituted with a primary amine group, and 4-carboxylicphenyl-boronic acid (CPBA), which is a glycated albumin-binding material, was fixed to the surface thereof, thus manufacturing a "tartrazine-encapsulated silica nanoparticle-boronic acid". Bromocresol green, which is a dye specifically binding to total albumin, was added thereto, thus manufacturing a reagent composition for measuring glycated albumin, the composition including the same.

Next, the filtered plasma sample was added to the "tartrazine-encapsulated silica nanoparticle-boronic acid" and "bromocresol green" to perform reaction, and was then injected into the absorption pad, followed by washing. Red (430 nm) and blue (630 nm) light sources were radiated on the absorption pad using the optical instrument to thus measure the optical reflectance of each of glycated albumin labeled with the "tartrazine-encapsulated silica nanoparticle-boronic acid" and total albumin labeled with bromocresol green. Thereby, it could be confirmed that the ratio of glycated albumin was capable of being measured simply and quickly.

Therefore, in an aspect, the present invention relates to a reagent composition for measuring glycated albumin, the composition including a dye specifically binding to albumin and a "dye-encapsulated silica nanoparticle-boronic acid" specifically binding to glycated albumin.

In the present invention, any dye specifically binding to total albumin may be used without particular limitation, as long as the dye is a dye that specifically reacts with albumin and glycated albumin. Examples thereof may include bromocresol green, which is blue and has an absorption wavelength band of 620 nm, or bromocresol purple, which is purple and has an absorption wavelength band of 580 nm at a physiologically neutral pH.

On the other hand, in the present invention, as the dye encapsulated in the silica nanoparticles, a yellow-colored dye or a red-colored dye that is complementary in color to the dye specifically binding to total albumin is used. The yellow-colored dye has an absorption wavelength of about 400 to 430 nm, and the red-colored dye has an absorption wavelength of about 500 to 530 nm. Examples of the yellow-colored dye may include tartrazine (425 nm), and examples of the red-colored dye may include red 80 (528 nm), without being limited thereto.

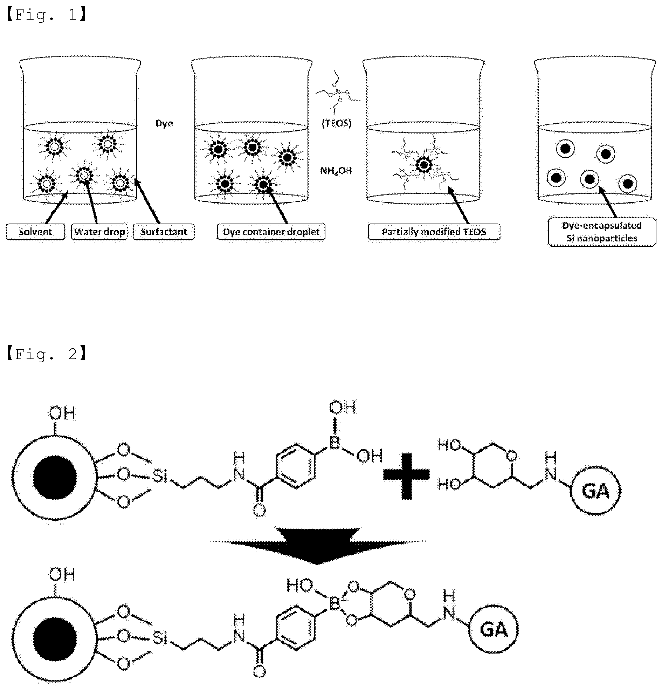

As shown in FIG. 1, the "dye-encapsulated silica nanoparticles" may be manufactured by adding a dye and silica to a mixture solution of water and a surfactant or a mixture solution of water and an organic solvent, followed by agitation and then addition of a basic catalyst.

The surfactant is not particularly limited, but triton x-100 or n-hexane may be used in the present invention. Examples of the silica may include tetraethyl orthosilicate or tetramethyl orthosilicate.

The basic catalyst is to promote the encapsulation of the dye by a silica precursor, which may promote the hydrolysis of water and the silica precursor. The ionized silica precursors react with each other to thus produce water and alcohol (ROH), which are connected to each other to thus form a silica network and grow.

Examples of the basic catalyst may include ammonium hydroxide, tetrapropylammonium chloride, tetrapropylammonium hydroxide, tetrabutylammonium bromide, tetrabutylammonium chloride, or tetrabutylammonium hydroxide.

In the case of the "dye-encapsulated silica nanoparticles", since the dye does not leak to the outside, stability and sensitivity may be increased, bio-toxicity may be low, and the functional groups on the surface thereof may be easily changed.

The diameter of the "dye-encapsulated silica nanoparticles" may be 10 to 500 nm, and preferably 30 to 100 nm, which makes it possible to maintain the inherent properties of the dye. When the diameter is less than 10 nm, it is difficult to perform the operation. When the diameter is more than 500 nm, since the thickness thereof is increased, the dye may appear cloudy.

As boronic acid derivatives for imparting selectivity for glycated albumin to the "dye-encapsulated silica nanoparticles", it is preferable to use 4-carboxylicphenyl boronic acid (CPBA) and 3-aminophenyl boronic acid (APBA). When the CPBA is used, the "dye-encapsulated silica nanoparticles" may be aminated. When the APBA is used, after the "dye-encapsulated silica nanoparticles" are carboxylated, conjugation may be performed via carbodiimide cross-coupling. Since the 4-carboxylicphenyl boronic acid (CPBA) has relatively higher thermal stability than the 3-aminophenyl boronic acid (APBA), it is preferable to use the CPBA.

As shown in FIG. 2, the "dye-encapsulated silica nanoparticle-boronic acid" may react with the cis-diol of glycated albumin. A plurality of dye molecules is encapsulated in the silica nanoparticles, so that the amount of light absorbed by one particle is larger than that absorbed by one dye molecule. Accordingly, the detection limit of glycated albumin may be improved.

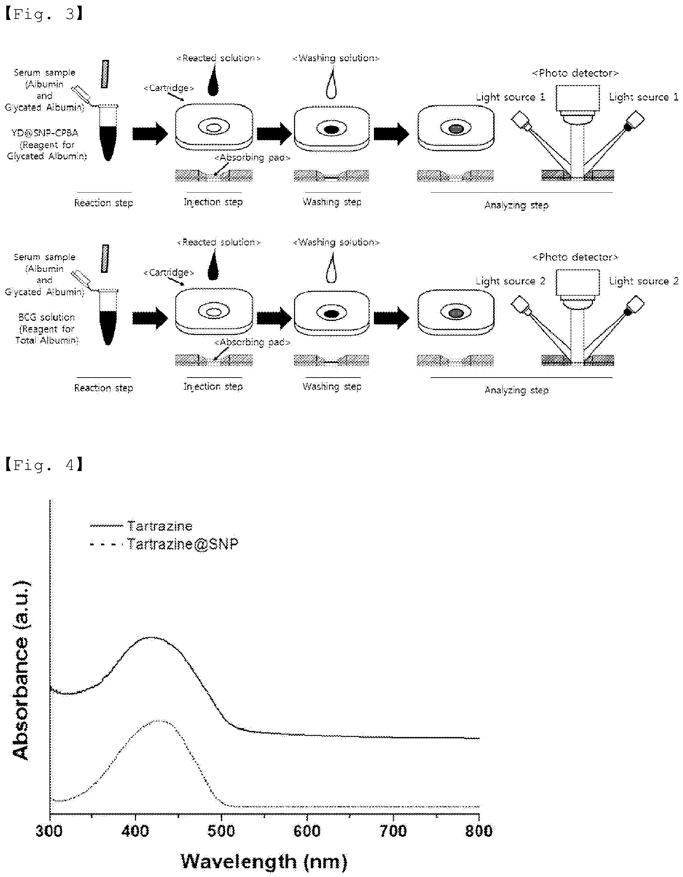

As shown in FIG. 3, blood or the plasma solution may be reacted with a "dye-encapsulated silica nanoparticle-boronic acid" capable of labeling glycated albumin, dropped on the cartridge, and washed, and the reflectance value of glycated albumin may then be recorded. Thereafter, the resultant substance may be reacted with a bromocresol green (BCG) solution capable of dyeing total albumin, dropped on the cartridge, and washed, and the reflectance value of total albumin may then be recorded. Thereby, the % value of glycated albumin may be calculated using the reflectance of total albumin relative to the reflectance of glycated albumin. After total albumin is measured, glycated albumin may be measured in order to calculate the % value of glycated albumin.

Accordingly, in another aspect, the present invention relates to a method of measuring glycated albumin. The method includes (a) introducing blood or a plasma solution into a reagent including a "dye-encapsulated silica nanoparticle-boronic acid" specifically binding to glycated albumin, followed by reaction, (b) injecting a reactant into an absorption pad of a cartridge, followed by washing with a washing liquid, (c) measuring the optical reflectance of the absorption pad using an optical instrument to measure the amount of glycated albumin, (d) introducing blood or a plasma solution into a reagent including a dye specifically binding to total albumin, followed by reaction, (e) injecting a reactant into an absorption pad of a cartridge, followed by washing with a washing liquid, (f) measuring the optical reflectance of the absorption pad using the optical instrument to measure the amount of total albumin, and (g) calculating a ratio of glycated albumin on the basis of the measured amounts of glycated albumin and total albumin.

In the present invention, any optical instrument may be used without particular limitation as long as the optical instrument can measure optical reflectance using optical properties. The optical instrument may radiate a wavelength of a dye (blue or purple) specifically binding to total albumin and a specific wavelength of the "dye(yellow or red)-encapsulated silica nanoparticle-boronic acid" as light sources (e.g.: blue (630 nm) and red (430 nm)) that can simultaneously emit predetermined wavelengths, and may measure the reflected optical signal using a photodiode detector (PD), thereby measuring the amounts of albumin and glycated albumin using an optical signal converter.

The ratio of glycated albumin may be obtained by calculating the amount of glycated albumin relative to the amount of total albumin using the following equation.

```
Ratio (%) of glycated albumin = glycated albumin /
total albumin
```

Generally, in the case when the ratio of glycated albumin is 16% or more, the case may be diagnosed as diabetes.

**Mode for Invention**

Hereinafter, the present invention will be described in more detail with reference to Examples. It is to be understood by those skilled in the art that these Examples are only for illustrating the present invention and that the scope of the present invention is not to be construed as being limited by these Examples.

Example 1: Manufacture of yellow dye-encapsulated silica nanoparticle-boronic acid (YD@SNP-CPBA)

1-1: Synthesis of yellow dye-encapsulated silica nanoparticles (YD@SNP)

135.0 ml of cyclohexane, 31.8 ml of Triton X-100, 32.4 ml of n-hexanol, 6.12 ml of 0.1 M tartrazine, and 2.7 ml of TEOS (tetraethyl orthosilicate) were added to a 1 L round bottom flask, and uniformly mixed for 1 hour using an agitator. 1.08 ml of 25~30% aqueous ammonia ($NH_4OH$) was added thereto and reacted at room temperature for 24 hours. 200 ml of ethanol was then added to terminate the reaction. Ethanol washing and DI washing were respectively performed four times and three times using a centrifuge at 3800 rpm for 15 minutes, followed by drying in an oven at 60°C.

1-2: Amination of yellow dye-encapsulated silica nanoparticles (YD@SNP)

In order to perform cross-coupling of the carboxyl groups of YD@SNP and CPBA, the hydroxyl group (-OH) on the surface of YD@SNP was substituted with a primary amine group. That is, 100 mg of YD@SNP was added to 100 ml of ethanol and dispersed for 30 minutes using an ultrasonic disperser. Then, 1 ml of APTES (3-aminopropyltriethoxysilane) was added to an agitator, followed by reaction at room temperature for 2 hours. After the reaction, ethanol washing and DI washing were respectively performed four times and three times using a centrifuge at 3800 rpm for 15 minutes, followed by drying in an oven at 60°C, thus manufacturing aminated YD@SNP (YD@SNP-$NH_2$).

1-3: Joining of aminated yellow dye-encapsulated silica nanoparticles (YD@SNP-$NH_2$) and CPBA

In order to provide binding ability to glycated albumin, according to a carbodiimide cross-coupling method using 1-ethyl-3[3-dimethylaminopropyl]carbodiimide hydrochloride (EDC), which is a cross-coupling agent connecting a carboxyl group and a primary amine group, 4-carboxylicphenyl-boronic acid (CPBA), which is a glycated albumin-binding material, was fixed on the surface of the aminated yellow dye-encapsulated silica nanoparticles (YD@SNP-$NH_2$).

That is, in order to activate the carboxyl functional group of CPBA in an environment from which light was blocked, 3.48 mM CPBA was dissolved in a 0.1 M MES (2-(N-morpholino)ethanesulfonic acid) buffer solution (pH 6.0), EDC having a final concentration of 1 mM was added thereto, and the reaction was allowed to progress for 30 minutes with agitation. Then, YD@SNP-NH$_2$ was added, followed by reaction in an agitator at room temperature for 10 to 20 hours.

After completion of the reaction, ethanol washing and DI washing were respectively performed four times and three times using a centrifuge at 3800 rpm for 15 minutes, followed by drying at room temperature or freeze-drying, thus manufacturing a yellow dye-encapsulated silica nanoparticle-boronic acid (YD@SNP-CPBA).

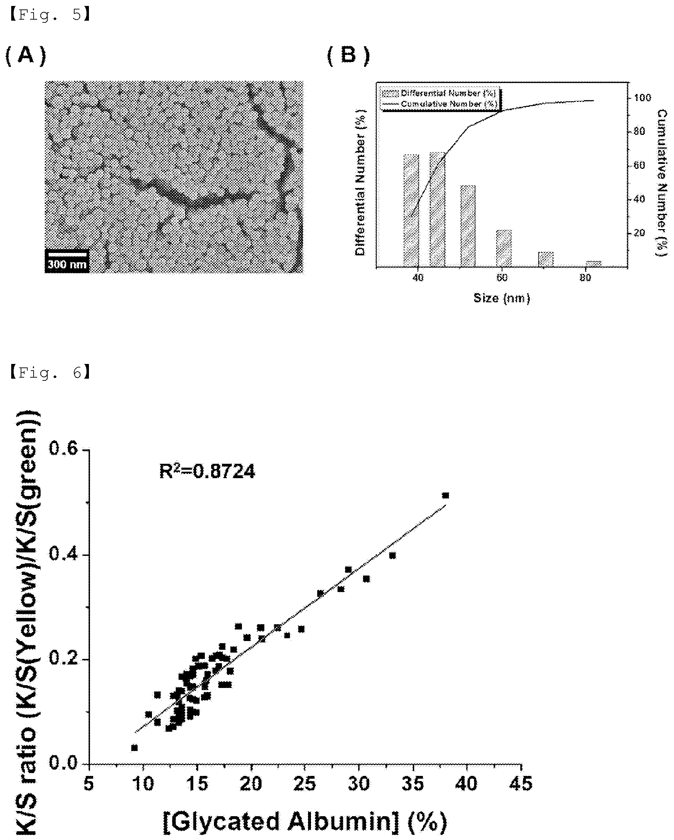

Each of the "tartrazine" dye used in the synthesis and the "dye-encapsulated silica nanoparticle-boronic acid" was diluted with deionized water (DI water), and measurement with UV/Vis spectroscopy was then performed. As a result, as shown in FIG. 4, it can be seen that the "dye-encapsulated silica nanoparticle-boronic acid" has an inherent absorption wavelength of the yellow dye even after the synthesis. With respect to this, analysis was performed using a scanning electron microscope and a dynamic light-scattering method. As a result, as shown in FIG. 5, it could be confirmed that nanoparticles having uniform morphology and a size of about 35 to 45 nm were synthesized.

Example 2: Measurement of glycated albumin using a reagent composition for measuring glycated albumin containing YD@SNP-CPBA and bromocresol green

2-1: Measurement of glycated albumin

200 μl of a reagent composition containing YD@SNP-CPBA manufactured in Example 1 (ZnCl$_2$, NaCl, MgCl$_2$, Triton X-100, NaN$_3$, glycine, HEPES, pH 8.1) was placed in a brown tube, and 5 μl of a plasma sample in which the % value of glycated albumin was measured using an Olympus AU 400 analyzer and a reference reagent (Asahi Kasei GA-L) was added thereto, followed by reaction for 2 minutes. 25 μl of the reaction solution was put on an absorption pad of a cartridge of an optical instrument (Epithod®616, DxGen) to be absorbed for 15 seconds, and 25 μl of a washing solution (morpholine, NaCl, Triton X-100, glycerol, and NaN$_3$ mixture solution) was added thereto, followed by washing for 15 seconds. Next, the optical reflectance of yellow glycated albumin on the cartridge was measured in the optical instrument (Epithod®616, DxGen).

2-2: Measurement of total albumin

200 μl of a reagent composition containing bromocresol green (Succinic acid, pH 5.5) was placed in a brown tube, and 5 μl of the same plasma sample was added thereto, followed by reaction for 2 minutes. 25 μl of the reaction solution was put on an absorption pad of a cartridge of an optical instrument Epithod®616, DxGen) to be absorbed for 15 seconds, and 25 μl of a washing solution (morpholine, NaCl, Triton X-100, glycerol, and NaN$_3$ mixture solution) was added thereto, followed by washing for 15 seconds. Next, the optical reflectance of blue total albumin on the cartridge was measured in the optical instrument (Epithod®616, DxGen).

2-3: Measurement of glycated albumin using K/S value

The % value of glycated albumin was determined by comparing the optical reflectance of glycated albumin measured in 2-1 and the optical reflectance of total albumin measured in 2-2. The % reflectance (%R) measured for each wavelength was converted into a K/S value, which is a quantitative index of how much of the coloring material is present on the surface thereof in use, and the formula for converting the % reflectance into the K/S value is as follows.

$$K/S = \frac{(1 - \%R)^2}{2 \times \%R}$$ (K = absorption coefficient, S = scattering coefficient)

Therefore, after the % reflectance value obtained by radiating the yellow light source representing the amount of glycated albumin and the % reflectance value obtained from the blue light source representing the amount of the total albumin were each substituted with the K/S value, the ratios thereof were calculated, thereby measuring the amount of glycated albumin.

As shown in FIG. 6, it could be seen that, as the ratio of glycated albumin contained in the blood is increased, the relative amount (K/S value) of the "dye-encapsulated silica nanoparticle-boronic acid" binding thereto is increased. Therefore, it was confirmed that the method of measuring glycated albumin according to the present invention is capable of being widely used to diagnose diabetes.

Although specific portions of the present invention have been described in detail above, those skilled in the art will appreciate that this specific description is only a preferred embodiment, and that the scope of the present invention is not limited thereby. Accordingly, the actual scope of the present invention will be defined by the appended claims and their equivalents.

**Industrial Applicability**

The reagent composition for measuring glycated albumin according to the present invention contains a dye that is used to distinguish total albumin and glycated albumin by labeling, so that glycated albumin may be measured in a simple manner using an optical analyzer merely by injecting a washing liquid into a measurement cartridge without any separation process. Accordingly, the reagent composition is capable of being widely used to diagnose diabetes.

**Claims**

1.  A reagent composition for measuring glycated albumin, the composition comprising:

    (a) a dye specifically binding to total albumin, and (b) a "dye-encapsulated silica nanoparticle-boronic acid" specifically binding to the glycated albumin.

2.  The reagent composition of claim 1, wherein the dye specifically binding to the total albumin is bromocresol green or bromocresol purple.

3.  The reagent composition of claim 1, wherein a dye encapsulated in silica nanoparticles is a yellow-colored dye or a red-colored dye.

4.  The reagent composition of claim 3, wherein the yellow-colored dye has an absorption wavelength of 400 to 430 nm and the red-colored dye has an absorption wavelength of 500 to 530 nm.

5.  The reagent composition of claim 3, wherein the yellow-colored dye is tartrazine and the red-colored dye is red 80.

6.  The reagent composition of claim 1, wherein "dye-encapsulated silica nanoparticles" are manufactured by adding a dye and silica to a mixture solution of water and a surfactant or a mixture solution of water and an organic solvent, followed by agitation and addition of a basic catalyst, and specifically bind to the glycated albumin.

7.  The reagent composition of claim 1, wherein a diameter of "dye-encapsulated silica nanoparticles" is 10 to 500 nm.

8.  The reagent composition of claim 1, wherein the "dye-encapsulated silica nanoparticle-boronic acid" is manufactured by aminating "dye-encapsulated silica nanoparticles", followed by conjugation with 4-carboxylicphenyl boronic acid (CPBA), or by carboxylating the "dye-encapsulated silica nanoparticles", followed by conjugation with 3-aminophenylboronic acid (APBA).

9.  A method of measuring glycated albumin, the method comprising:

    (a) introducing blood or a plasma solution into a reagent including a "dye-encapsulated silica nanoparticle-boronic acid" specifically binding to the glycated albumin, followed by reaction;
    (b) injecting a reactant into an absorption pad of a cartridge, followed by washing with a washing liquid;
    (c) measuring an optical reflectance of the absorption pad using an optical instrument to measure an amount of the glycated albumin;
    (d) introducing the blood or the plasma solution into a reagent including a dye specifically binding to total albumin, followed by reaction;
    (e) injecting a reactant into the absorption pad of the cartridge, followed by washing with the washing liquid;
    (f) measuring an optical reflectance of the absorption pad using the optical instrument to measure an amount of the total albumin; and
    (g) calculating a ratio of the glycated albumin on a basis of measured amounts of the glycated albumin and the total albumin.

10. The method of claim 9, wherein the optical instrument simultaneously radiates a wavelength of the dye specifically binding to the total albumin and a specific wavelength of the "dye-encapsulated silica nanoparticle-boronic acid" as light sources, thus measuring the optical reflectance.

11. The method of claim 9, wherein diabetes is diagnosed according to the ratio of the glycated albumin.

【Fig. 1】

【Fig. 2】

【Fig. 3】

【Fig. 4】

【Fig. 5】

(A)

300 nm

(B)

【Fig. 6】

# EP 3 517 963 A1

## INTERNATIONAL SEARCH REPORT

| International application No. |
|---|
| PCT/KR2017/010499 |

### A. CLASSIFICATION OF SUBJECT MATTER

*G01N 33/68(2006.01)i, G01N 33/52(2006.01)i, G01N 33/543(2006.01)i, G01N 21/17(2006.01)i*

According to International Patent Classification (IPC) or to both national classification and IPC

### B. FIELDS SEARCHED

Minimum documentation searched (classification system followed by classification symbols)

G01N 33/68; B82B 3/00; G01N 33/558; G01N 33/483; G01N 21/31; G01N 21/17; G01N 33/72; G01N 1/10; C01B 33/12; G01N 33/52; G01N 33/543

Documentation searched other than minimum documentation to the extent that such documents are included in the fields searched
Korean Utility models and applications for Utility models: IPC as above
Japanese Utility models and applications for Utility models: IPC as above

Electronic data base consulted during the international search (name of data base and, where practicable, search terms used)

eKOMPASS (KIPO internal) & Keywords: glycated albumin, dye, encapsulation, silicon nano particle, boronic acid, light source, wavelength, reflectance

### C. DOCUMENTS CONSIDERED TO BE RELEVANT

| Category* | Citation of document, with indication, where appropriate, of the relevant passages | Relevant to claim No. |
|---|---|---|
| Y | US 5919708 A (SUNDREHAGEN, Erling) 06 July 1999<br>See claims 1-3 and 13-14; column 1, lines 27-32; column 8, lines 1-6; column 15, lines 7-20; column 21, lines 20-22; column 23, lines 9-30. | 1-11 |
| Y | KR 10-2008-0023280 A (SAMSUNG ELECTRONICS CO., LTD. et al.) 13 March 2008<br>See claim 1; paragraphs [0009] and [0017]-[0019] of "The detailed description of the invention"; figure 2. | 1-11 |
| Y | WO 2014-033258 A1 (AXIS-SHIELD ASA.) 06 March 2014<br>See page 9, third paragraph; pages 13-15. | 10 |
| A | WANG, Jiasheng et al., "Silica-based Nanocomposites via Reverse Microemulsions: Classifications, Preparations, and Applications", Nanoscale, 2014, vol. 6, no. 9, pages 4418-4437, (View Article Online Version, pages 1-20)<br>See the entire document. | 1-11 |
| A | KR 10-2010-0137851 A (ALL MEDICUS CO., LTD. et al.) 31 December 2010<br>See claims 1-4. | 1-11 |
| A | KR 10-2016-0015495 A (K-MAC) 15 February 2016<br>See claims 1-10. | 1-11 |

| ☐ Further documents are listed in the continuation of Box C. | ☒ See patent family annex. |
|---|---|

\* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 15 JANUARY 2018 (15.01.2018) | **15 JANUARY 2018 (15.01.2018)** |

| Name and mailing address of the ISA/KR | Authorized officer |
|---|---|
| Korean Intellectual Property Office<br>Government Complex-Daejeon, 189 Seonsa-ro, Daejeon 302-701, Republic of Korea<br>Facsimile No. +82-42-481-8578 | Telephone No. |

Form PCT/ISA/210 (second sheet) (January 2015)

13

INTERNATIONAL SEARCH REPORT
Information on patent family members

International application No.

**PCT/KR2017/010499**

| Patent document cited in search report | Publication date | Patent family member | Publication date |
|---|---|---|---|
| US 5919708 A | 06/07/1999 | AT  112635  T | 15/10/1994 |
| | | AU 8866291 A | 11/06/1992 |
| | | CA 2096250 A1 | 15/05/1992 |
| | | CA 2096250 C | 24/09/2002 |
| | | DE 69104491 T2 | 16/02/1995 |
| | | DK 0557350 T3 | 07/11/1994 |
| | | EP 0557350 A1 | 01/09/1993 |
| | | EP 0557350 B1 | 05/10/1994 |
| | | ES 2061273 T3 | 01/12/1994 |
| | | FI 104519 B | 15/02/2000 |
| | | FI 932150 A | 03/06/1993 |
| | | JP 06-502244 A | 10/03/1994 |
| | | JP 2643027 B2 | 20/08/1997 |
| | | NO 311385 B1 | 19/11/2001 |
| | | NO 931747 A | 14/07/1993 |
| | | US 5506144 A | 09/04/1996 |
| | | WO 92-08984 A1 | 29/05/1992 |
| KR 10-2008-0023280 A | 13/03/2008 | KR 10-0818462 B1 | 01/04/2008 |
| | | US 2008-0063868 A1 | 13/03/2008 |
| WO 2014-033258 A1 | 06/03/2014 | NONE | |
| KR 10-2010-0137851 A | 31/12/2010 | KR 10-1042043 B1 | 16/06/2011 |
| KR 10-2016-0015495 A | 15/02/2016 | KR 10-1623372 B1 | 24/05/2016 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7871789 B
- US 6008006 A
- US 8507223 B
- CN 104673878
- CN 104614459
- US 5223392 A
- US 5908925 A
- EP 0657470 A
- EP 0257421 A
- CN 103554256
- US 9128085 B
- US 20060270060 A
- US 20080227210 A
- US 20100167306 A
- US 5470759 A
- US 7659107 B
- US 20140170766 A
- US 20140335630 A
- US 5631364 A
- US 7374943 B
- US 2014033258 A
- US 5589393 A
- US 8557590 B
- KR 1128037

**Non-patent literature cited in the description**

- **IKEDA et al.** *Clin Chem.,* 1998, vol. 44 (2), 256-263